# EUROPEAN PATENT SPECIFICATION

(11) **EP 4 356 132 B1**
(45) Date of publication and mention of the grant of the patent: **01.10.2025**
(21) Application number: 22733947.0
(22) Date of filing: 15.06.2022
(51) Int. Cl.: G01N 33/543, C12Q 1/6804, B82Y 15/00, B82Y 30/00

(54) **METHOD FOR IMMUNOSENSING ON A LIPID LAYER**
VERFAHREN ZUM IMMUNOSENSING AUF EINER LIPIDSCHICHT
PROCÉDÉ D'IMMUNODÉTECTION SUR UNE COUCHE LIPIDIQUE

(30) Priority: 17.06.2021 EP 21179987
(43) Date of publication of application: 24.04.2024
(73) Proprietor: F. Hoffmann-La Roche AG, 4070 Basel (CH); Roche Diagnostics GmbH, 68305 Mannheim (DE)
(72) Inventor: STENGELE, Nikolaus-Peter, 82377 Penzberg (DE)
(74) Representative: Altmann Stößel Dick Patentanwälte PartG mbB
(86) International application number: PCT/EP2022/066335
(87) International publication number: WO 2022/263523

(56) References cited:
- WO-A1-2015/175856
- WO-A1-2016/161402
- WO-A1-2018/067878
- WO-A1-2020/023503
- WO-A1-2020/077309
- WO-A1-2020/180645
- WO-A2-2007/024676
- WO-A2-2007/092909
- US-A1- 2007 154 881
- US-A1- 2014 272 939

## Description

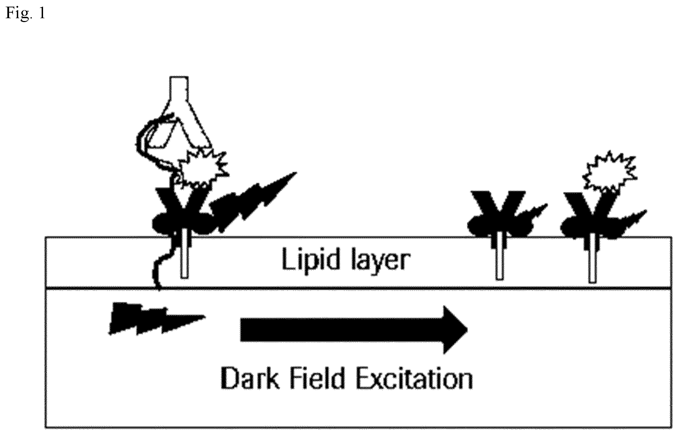

Prior art document, WO 2015/175856 A1 discloses a method of detection comprising a capture reagent and a detection reagent. The capture reagent is defined as "(i) a capture reagent on a surface comprising the capture reagent for the analyte, and an anchoring reagent: and (ii) a detection reagent for the analyte that is linked to a nucleic acid probe", sec c.f. page 2. lines 18-19 and Fig. 1 (a). The method of detection is based on an extension process resulting in an extended sequence that is connected to the detection reagent and capable of binding the anchoring reagent; sec cf. page 3. lines 22-24: "extending the probe to form an extended sequence comprising an anchoring region that binds the anchoring reagent: binding the extended sequence to the anchoring reagent: and measuring the amount of extended sequence bound to the surface." Further, documents WO 2015/175856 A1, WO 2020/180645 A1 and US 2014/272939 A1 disclose a detection agent linked to a nucleic acid probe. The analyte binds to the immobilized capture agent and the detection agent. A sandwich complex is formed comprising the analyte, the surface immobilized capture agent and the detection agent linked to the nucleic acid probe. Then the nucleic acid probe may be extended to form an anchoring region. The anchoring region may bind to the anchoring reagent as depicted in e.g. Fig. 1 (a) of WO 2015/175856 A1, WO 2020/180645 A1 and US 2014/272939. Thus, according to the teachings of WO 2015/175856 A1, WO 2020/180645 A1 and US 2014/272939 the binding of anchoring reagent and anchoring region merely occurs after complex formation of the capture agent, the analyte and the detection reagent. WO 2007/024676 A2 relates to methods for detecting at least one target analyte in a sample. The methods comprise using a target analyte with at least two binding sites; the target analyte may be bound by two different types of capture probes and optionally a type of detector probe (sec page 4. lines 5-18). A first capture probe is provided on a substrate. i.e. may be interpreted as being immobilized on a support. However, none of the disclosed probes is anchored in an anchor layer, neither the further capture probe nor the detector probe.

The present invention relates to diagnostic test and technology. In particular, it relates to the present invention relates to a method for determining an analyte suspected to be present in a sample comprising contacting said sample with a sensor element comprising i) an anchor layer which is present on a solid support, ii) a first binding agent which is capable of specifically binding to the analyte, which is anchored in the anchor layer and which comprises at least one detectable label, and iii) a second binding agent which is capable of specifically binding to the analyte when bound to the first binding agent and which is immobilized on the solid support, for a time and under conditions which allow for specific binding of the analyte suspected to be present in the sample to the first binding agent and specific binding of the second binding agent to the analyte bound to the first binding agent and detecting the formation of the complex of first binding agent, analyte and second binding agent whereby the analyte is determined. Moreover, provided is a device for determining an analyte suspected to be present in a sample and the use thereof for determining an analyte suspected to be present in a sample in said sample. Moreover, the present invention contemplates a kit for determining an analyte suspected to be present in a sample.

Immunoassays are widely used for various diagnostic purposes. Several setups have been developed for immunoassays. One of the most popular immunoassays is the enzyme-linked sorbent immunoassay (ELISA).

In ELISA, a liquid sample containing an analyte of interest or suspected to contain an analyte of interest is applied onto a stationary solid phase with special binding properties due to the presence of antibodies or antibody-like molecules such as aptamers. After sample application, multiple reagents are sequentially added, incubated, and washed away in order to carry out and stop an analytical detection reaction. After carrying out all these steps, physical or chemical properties in the setup, usually the liquid phase, are changed that can be detected. Typically, optical change such as color development by the product of an enzymatic reaction will happen in the final liquid phase. These changes correlate to the presence or abundance of the analyte of interest present in the investigated sample. The typically quantitative read-out is usually based on detection of intensity of transmitted light by spectrophotometry, which involves quantitation of transmission of some specific wavelength of light through the liquid. The sensitivity of detection depends on amplification of the signal during the analytic reactions. Since enzyme reactions are very well known amplification processes, the signal is generated by enzymes which are linked to the detection reagents in fixed proportions to allow accurate quantification.

For an ELISA setup, the analyte binding agent, e.g., an antibody, is immobilized on a solid phase such as a solid support structure. Usually, the antibody is coated and dried onto the transparent bottom and sometimes also side wall of a well in an analytic multi-well plate or in an analytic vial. However, nanoparticles or other beads can also be used as solid phases for ELISAs.

For research and diagnostic, ELISAs are often used in the format of so-called sandwich ELISAs. In a sandwich format, an immobilized capture antibody is used to capture, i.e. specifically bind to, an analyte present in a sample applied to said immobilized antibody. After the capture antibody has specifically bound to the analyte, the sample material is washed away. In a subsequent step the analyte bound to the immobilized antibody is incubated with a detection antibody that specifically binds to the analyte or the complex of analyte and capture antibody. The detection antibody typically contains a detectable linker or an adaptor molecule which allows for attracting such detectable labels from a solution.

However, in light of the fragile immune complexes which are required for signal generation and the various components used for such an ELISA in the sandwich format, there are many possibilities for undesired binding of detection antibodies and thus for the generation of false positive signals. Therefore, sandwich ELISAs used for research often need validation because of the risk of false positive results. Moreover, there are limitations with respect to sensitivity and specificity due to the various drawbacks of such a fragile multi-component assay format.

The technical problem underlying the present invention may be seen as the provision of means and methods for complying with the aforementioned needs. The technical problem is solved by the embodiments characterized in the claims and herein below.

Thus, the present invention relates to a method for determining an analyte suspected to be present in a sample comprising:
(a) contacting said sample with a sensor element comprising:
   (i) an anchor layer which is present on a solid support;
   (ii) a first binding agent which is capable of specifically binding to the analyte, which is anchored in the anchor layer and which comprises at least one detectable label; and
   (iii) a second binding agent which is capable of specifically binding to the analyte when bound to the first binding agent and which is immobilized on the solid support;
   for a time and under conditions which allow for specific binding of the analyte suspected to be present in the sample to the first binding agent and specific binding of the second binding agent to the analyte bound to the first binding agent; and
(b) detecting the formation of the complex of first binding agent, analyte and second binding agent whereby the analyte is determined.

It is to be understood that in the specification and in the claims, "a" or "an" can mean one or more, depending upon the context in which it is used. Thus, for example, reference to "an" item can mean that at least one item can be utilized.

As used in the following, the terms "have", "comprise" or "include" are meant to have a non-limiting meaning or a limiting meaning. Thus, having a limiting meaning these terms may refer to a situation in which, besides the feature introduced by these terms, no other features are present in an embodiment described, i.e. the terms have a limiting meaning in the sense of "consisting of" or "essentially consisting of". Having a non-limiting meaning, the terms refer to a situation where besides the feature introduced by these terms, one or more other features are present in an embodiment described.

Further, as used in the following, the terms "preferably", "more preferably", "most preferably", "particularly", "more particularly", "typically", and "more typically" or similar terms are used in conjunction with additional or alternative features, without restricting alternative possibilities.

Further, it will be understood that the term "at least one" as used herein means that one or more of the items referred to following the term may be used in accordance with the invention. For example, if the term indicates that at least one item shall be used this may be understood as one item or more than one item, i.e. two, three, four, five or any other number. Depending on the item the term refers to the skilled person understands as to what upper limit the term may refer, if any.

The method according to the present invention may consist of the aforementioned steps (a) and (b) or may comprise further steps, such as pretreating or isolating the sample prior to step (a) and/or after step (b) further one or more steps of evaluating the determined analyte, e.g., by comparing it to references in order to provide a diagnostic, prognostic, environmental-relevant, agricultural-relevant or analytically-relevant conclusion depending on the aim of the determination of the analyte.

The term "determining" as used herein encompasses any kind of qualitative or quantitative determinations of the analyte. A qualitative determination aims at determining the presence or the absence of the analyte in the sample whereas quantitative determination aims at determining the amount of the analyte. The quantitative determination, i.e. the determination of the amount, includes determining the absolute amount (e.g. the total amount by weight or number of molecules present in the sample) or a relative amount (e.g., an amount relative to the sample volume (concentration) or a classification such as a score (e.g., "high amount", "low amount" and the like). Typically, determining an analyte comprises determining the presence, absence or amount of said analyte.

The term "analyte" as referred to herein relates to any type of molecule or agent which is suitable for being determined by the method of the invention. It will be understood that such a molecule or agent may have a size and/or structure that allows binding of the first and second binding agents referred to herein. Moreover, there may be upper size limitations as well since the first and second binding agents as well as the linking agent must be capable of exert their functions as described elsewhere herein in detail. Typically, the analyte referred to herein is a biological molecule such as a protein, a peptide, a nucleic acid, e.g., a DNA or RNA or a small molecule such as a lipid or metabolite, a polyketide including, e.g., flavonoids and isoflavonoids, an isoprenoid including, e.g., terpenes, sterols, steroids, carotenoids, xanthophylls, a carbohydrate, a phenylpropanoide, an alcaloide, a benzenoide, an indole, a porphyrine, a hormone, a vitamin, a cofactor, a lignin, a glucosinolate, a purine, a pyrimidine, a nucleoside, a nucleotide, an alcohol, an alkane, an alkene, an alkine, an aromatic compound, a ketone, an aldehyde, a carboxylic acid, a ester, an amine, an imine, an amide, a cyanide, an amino acid, a thiol, a thioester, a phosphate ester, a sulfate ester, a thioether, a sulfoxide or an ether. The small molecule may be, e.g., a toxin. However, the method of the invention can also be used for determining viruses or even bacterial cells as analytes. Analytes to be determined by the present invention may also be molecules which are present in environmental samples and which may be useful as indicators for, e.g., environmental pollution, agriculture or other environmental conditions. Typically, said analyte is a protein, peptide, virus, bacterial cell or small molecule, preferably, small molecule toxin.

The term "sample" as used herein relates to any part or aliquot of a composition comprising or suspected to comprise the analyte to be determined. Such a sample may be a biological sample, typically, isolated from an organism, such as a body fluid or biopsy sample, or may be a composition comprising organism such as a cultured cells. Typically, said biological sample is investigated by the method of the invention for medical purposes, such as diagnosing or predicting a disease or medical condition. Moreover, the sample may be an environmental sample or an artificial sample. An environmental sample may be derived from any non-biological, natural source, e.g., environmentally occurring solutions such as water or from compositions such as soil. An artificial sample may be a sample obtained from an artificial source, e.g., a product composition which may be manufactured or an intermediate composition which may occur during a manufacturing process for a product. Such artificial samples may be investigated, e.g., for quality control purposes or for determining the amount of specific ingredients. Typically, said sample in accordance with the method of the present invention is a biological sample, preferably, a body fluid or biopsy sample.

The term "sensor element" as used in accordance with the present invention comprises a solid support having a surface which is covered by an anchor layer. Moreover, the sensor element shall further comprise a molecular arrangement as described above, i.e. a first binding agent which is capable of specifically binding to the analyte, which is anchored in the anchor layer and which comprises at least one detectable label and a second binding agent which is capable of specifically binding to the analyte when bound to the first binding agent and which is immobilized on the solid support.

The term "anchor layer" as referred to herein encompasses any molecular layer that is capable of associating molecules and, in particular, the first binding agent according to the invention. The association of the first binding agent referred to in accordance with the present invention shall allow molecular movements of the first binding agent or any anchoring molecule attached thereto within the anchor layer or on its surface. The said anchor layer is, typically, a lipid layer or a lipid bi-layer. The skilled artisan is well aware of how a solid support can be covered by such an anchor layer and which lipids can be used to create a lipid layer or lipid bi-layer. Typical lipid layers or lipid bi-layers suitable for use as an anchor layer according to the invention may include for example a phospholipid layer or phospholipid bi-layer. Said phospholipid layer or phospholipid bi-layer may particularly comprise one or more phosphatidylcholine(s), such as 1-oleoyl-2-palmitoyl-phosphatidylcholine, 1,2-dioleoyl-sn-glycero-3-phosphocholine and/or 1,2-dioleoyl-sn-glycero-3-[(N-(5-amino-1-carboxypentyl)iminodiacetic acid)succinyl], or a mixture of one or more phosphatidylcholine(s) and cholesterol. Moreover, supported lipid bilayers and tethered bilayer lipid membranes may find use as an anchor layer according to the invention. These are commonly used model lipid bilayers known in the art.

The term "solid support" as used herein relates to a solid composition of matter which can serve as a basis for immobilizing molecules and, in particular, the second binding agent. A solid support may comprise inorganic or organic compounds or both. Typically, suitable inorganic compounds for a solid support may be selected from the group consisting of: silica, porous glass, aluminosilicates, borosilicates, metal oxides (e.g. aluminum oxide, iron oxide, nickel oxide) and clay containing one or more of these. The solid support may also comprise conductive compounds such as a metal or graphite. Preferably, such a solid support may be or may comprise an electrode, a magnetic tunnel junction, a semiconductor, a plasmonic resonator or any kind of electrical circuit arrangement. Alternatively, the solid support may comprise an organic compound such as a cross-linked polymer. Non-limiting examples of a suitable cross-linked polymer may be selected from the group consisting of: polyamide, polyether, polystyrene and mixtures thereof. The skilled artisan is well aware of how to select a suitable solid support based on the kind of sample to investigated, the method envisaged for detection of the analyte, the kind of detectable label to be used for detecting the analyte and/or the kind of second binding agent used for the sensor element.

The sensor shall further comprise a first binding agent which is capable of specifically binding to the analyte. Said first binding agent shall be anchored in the anchor layer. Moreover, it shall comprises at least one detectable label.

The term "first binding agent" referred to herein relates to a molecule which is capable of specifically binding to the analyte, i.e. a molecule which does not bind to and, thus, does not cross-react with other molecules than the analyte suspected to be present in the sample. Specific binding, in principle, can be tested by techniques well known in the art including screening assays for identifying agents specifically binding an analyte from libraries comprising different candidate agents.

Molecules which may be used, preferably, as first binding agents being capable of specifically binding a desired analyte may be antibodies. Antibodies as binding agents as meant in accordance with the present invention encompass to all types of antibodies which, preferably, specifically binds to the analyte. Preferably, an antibody of the present invention may be a monoclonal antibody, a polyclonal antibody, a single chain antibody, a chimeric antibody or any fragment of such antibodies being still capable of specifically binding to the analyte. Such fragments comprised by the term antibody as used herein encompass a bispecific antibody, a synthetic antibody, an Fab, F(ab)₂ Fv or scFv fragment or a chemically modified derivative of any of these antibody fragments. Antibodies or fragments thereof, in general, which specifically bind to a desired analyte can be obtained by using methods which are described, e.g., in Harlow and Lane "Antibodies, A Laboratory Manual", CSH Press, Cold Spring Harbor, 1988. Monoclonal antibodies can be prepared by the techniques which comprise the fusion of mouse myeloma cells to spleen cells derived from immunized mammals and, preferably, immunized mice (Köhler 1975, Nature 256, 495, and Galfré 1981, Meth. Enzymol. 73,3). The skilled person is well aware of how specific binding can be tested by techniques well known in the art, such as immunological or biochemical techniques including immunoassays, cell sorting or Western blotting or plasmon surface resonance measurements.

Further, molecules which may be used, preferably, as first binding agents being capable of specifically binding a desired analyte may be aptamers. An aptamer as a binding agent in accordance with the present invention may be an oligonucleic acid or peptide molecule that binds to a specific target analyte (Ellington 1990, Nature 346 (6287): 818-22). Bock 1992, Nature 355 (6360): 564-6). Oligonucleic acid aptamers are engineered through repeated rounds of selection or the so-called systematic evolution of ligands by exponential enrichment (SELEX technology). Peptide aptamers usually comprise of a variable peptide loop attached at both ends to a protein scaffold. This double structural constraint shall increase the binding affinity of the peptide aptamer into the nanomolar range. Said variable peptide loop length is, preferably, composed of ten to twenty amino acids, and the scaffold may be any protein having improved solubility and compacity properties, such as thioredoxin-A. Peptide aptamer selection can be made using different systems including, e.g., the yeast two-hybrid system (see e.g., Hoppe-Seyler 2000, J Mol Med. 78 (8): 426-30). Also encompassed according to the present invention are any fragments of said aptamers that are still capable of specifically binding to the analyte. Said fragments can be used in isolated form or may be part of fusion molecules, i.e. molecules comprising said aptamer fragment as well as other parts such as linker moieties or adapter molecules. The skilled person is well aware of how specific binding can be tested by techniques well known in the art, such as plasmon surface resonance measurements.

Molecules which may be used, preferably, as first binding agents being capable of specifically binding a desired analyte may also be receptor molecules. A receptor molecule as a binding agent as referred to in accordance with the present invention is, typically, a protein that specifically binds to a ligand and which become activated upon ligand binding to exert its biological function. Such a receptor molecule or a fragment thereof being still capable of specifically binding to the ligand may also be used as a binding agent in accordance with the present invention for the ligand as analyte or a molecule which is derived from the ligand but still capable of being bound by the receptor molecule or fragment thereof such as antagonistically or agonistically acting variants of a ligand. Preferably, the receptor molecule envisaged as a binding agent in accordance with the present invention may be a transmembrane type receptor protein, such as a G-protein coupled receptor, e.g., a metabolic receptor, an enzyme linked receptor such as a receptor tyrosine kinase, e.g., a growth factor receptor, an immunological receptor, such as a virus receptor, a cell surface antigen, a T cell receptors, e.g., CD4, CD3 or CD8, or a MHC protein, a cell adhesion molecule, such as an integrin, cadherin, selectin or syndecan, a neuronal receptor or a pathogen receptor such as Toll-like receptors. The receptor molecule may also be a nuclear receptor protein such as a nuclear hormone receptor, e.g., the glucocorticoid receptor, retinoic acid receptor or thyroid hormone receptor.

The skilled person is well aware of receptor molecules or fragments thereof which specifically bind to an analyte to be determined or fragments thereof. Moreover, specific binding can be tested by techniques well known in the art, such as plasmon surface resonance measurements.

Yet, molecules which may be used, preferably, as first binding agents being capable of specifically binding a desired analyte may also be ligand molecules. A ligand molecule as a binding agent as referred to in accordance with the present invention is, typically, a protein or peptide that specifically binds to a receptor molecule and which activates upon receptor binding the said receptor molecule. Such a ligand molecule or a fragment thereof being still capable of specifically binding to the receptor molecule may also be used as a binding agent in accordance with the present invention for the receptor as analyte or a molecule which is derived from the receptor molecule but still capable of being bound by the ligand molecule or fragment thereof such as soluble variants of a receptor. Moreover, a ligand may also be any antigen which may be used to determine a certain antibody in a sample of an organism. Preferably, the ligand molecule envisaged as a binding agent in accordance with the present invention may be a peptide hormone, a neurotransmitter, a growth factor, such as angiopoietin, a BMP, a neutrotrophic factor, EGF, an epiphrin, EPO, a FGF, a GDNF, a GDF, insulin or an insulin-like growth factor, a TGF, a neutrophin, a VEGF, a cytokine, such as an interleukin, interferon, lymphokine, monokine, colony stimulating factor or chemokine, a extracellular matrix protein such as fibronectin, vitronectin, collagen, ankyrin or laminin, or the like. The skilled person is well aware of ligand molecules or fragments thereof which specifically bind to an analyte to be determined or fragments thereof. Moreover, specific binding can be tested by techniques well known in the art, such as plasmon surface resonance measurements.

Further preferably, the first binding agent may be a Designed Ankyrin Repeat Protein (DARPin). DARPins are genetically engineered antibody mimetic proteins which can be designed to achieve highly specific and high-affinity target protein binding. They are derived from natural ankyrin repeat proteins. Typically, DARPins comprise at least three repeat modules, of which the most N- and the most C-terminal modules (also referred to as "caps") protect the hydrophobic core of the protein (Binz 2003, Journal of Molecular Biology. 332 (2): 489-503).

Typically, said first binding agent is selected from the group consisting of: an antibody or fragment thereof, an aptamer, a receptor molecule or fragment thereof, and a ligand molecule or fragment thereof. More typically, it is an antibody or fragment thereof or an aptamer.

Moreover, the first binding agent in accordance with the present invention shall be anchored in the anchor layer. An anchored first binding agent as referred to in accordance with the present invention shall, typically, be associated with or be within the anchor layer. Accordingly, it is, typically, limited in its movements to essentially in two dimensions in space.

Preferably, said first binding agent is anchored in the anchor layer via an anchoring molecule, more preferably, a lipid. Suitable lipids for anchoring a first binding agent according to the present invention depend on the nature of the anchor layer and can be chosen by the skilled artisan without further ado, The anchoring molecule may be typically linked to at least one, preferably, a plurality of, first binding agent via a linker molecule. Suitable linker molecules allow for attaching many first binding agents and at least one anchoring molecules. Preferably, a suitable linker molecule may be a cyclo-DNA molecule. Thus, a plurality of first binding agents may be anchored to the anchor layer via the same anchoring molecule.

The term "detectable label" as used herein relates to molecules which exhibit physical or chemical properties which can be detected in the settings of the method of the present invention. Preferably, said properties are optically detectable properties such as emission of radiation, chemiluminescence, fluorescence or FRET, electromagnetic properties such as interference with electrical or magnetic fields, detectable radioactivity or chemical properties such as capability of performing or catalyzing a certain chemical reaction. The detectable label may be reversibly or permanently bound to the first binding agent. To this end, the detectable label may be a molecule which is capable of reversibly binding to the first binding agent or it may be a molecule or moiety which is already part of the said first binding agent. Typical detectable labels include gold particles, latex beads, acridan ester, luminol, ruthenium, enzymatically active labels, magnetic labels, radioactive labels or fluorescent labels. Enzymatically active labels include horseradish peroxidase, alkaline phosphatase, beta-Galactosidase or Luciferase. Suitable substrates for detection of such enzymatically detectable labels include di-amino-benzidine (DAB), 3,3'-5,5'-tetramethylbenzidine, NBT-BCIP (4-nitro blue tetrazolium chloride and 5-bromo-4-chloro-3-indolyl-phosphate. A suitable enzyme-substrate combination may result in a colored reaction product, fluorescence or chemiluminescence which can be measured according to methods known in the art, e.g., by using a light-sensitive film or a suitable camera system. Typical fluorescent labels include fluorescent proteins, such as GFP, RFP, YFP, BFP or variants thereof, Cy3, Cy5, Texas Red, Fluorescein, and the Alexa dyes, e.g., Alexa 568. Also the use of quantum dots as fluorescent labels is contemplated. Typical radioactive labels include 35S, 125I, 32P, 33P and the like. A radioactive label can be detected by any method known and appropriate, e.g. a light-sensitive film or a phosphor imager. Typical magnetic labels comprise iron-platinum nanoparticles, iron nanoparticles, nickel nanoparticles or cobalt nanoparticles. Typically, said detectable label is selected from the group consisting of: fluorescent labels, chemiluminescent label, radioactive labels, magnetic labels, and electrochemical labels. Typically, said detectable label comprises a linker which can be covalently linked by the linking agent to the solid support.

The first binding agent may comprise the at least one detectable label as a part of the molecule. Alternatively, the at least one detectable label may be linked to the first binding molecule by a linker. Permanent linkage as well as reversible linkage are envisaged in accordance with the present invention. Reversible linkage may be achieved, e.g., by using biotin-streptavidin based molecular adapter systems well known in the art.

The sensor shall also comprise a second binding agent which is capable of specifically binding to the analyte when bound to the first binding agent. The said second binding agent shall be immobilized on the solid support.

The term "second binding agent" which is capable of specifically binding to the analyte relates to a molecule which is capable of specifically binding to the analyte or the analyte when bound to the first binding agent, i.e. a molecule which does not bind to and, thus, does not cross-react with other molecules than the analyte suspected to be present in the sample or the complex of the analyte and the first binding agent. Typically, said second binding agent is selected from the group consisting of: an antibody or fragment thereof, an aptamer, a receptor molecule or fragment thereof, and a ligand molecule or fragment thereof. More typically, it is an antibody or fragment thereof or an aptamer. Typically, the second binding agent is from the same of the aforementioned molecule classes as the first binding agent. The definitions for an antibody or fragment thereof, an aptamer, a receptor molecule or fragment thereof, and a ligand molecule or fragment thereof made in accordance with the first binding agent apply mutatis mutandis for the second binding agent.

The second binding agent shall be immobilized on the solid support. Immobilization of said second binding agent, preferably, is a permanent immobilization. Suitable techniques for immobilizing the second binding agent to the solid support depend on the nature of the solid support and/or the nature of the second binding agent and are well known to the skilled artisan. It will be understood that upon immobilization the second binding agent shall not be capable of moving its position within or outside the anchor layer. However, the second binding agent shall be capable of carrying out bending and other molecular movements or conformational changes required to bind to the analyte or to a first binding agent bound to the analyte.

Moreover, the interaction of the second binding agent and the analyte and first binding agent shall be, preferably, a reversible interaction. It is envisaged that the association rate is typically larger than the dissociation rate such that complexes of first binding agent, analyte and second binding agent remain stable over a certain time allowing for their determination by detection the presence or abundance of the at least one detectable label within proximity to the position of the second binding agent during said time window.

Typically, the amount of said first binding agent exceeds the amount of said second binding agent by a factor between 10 and 100, between 20 and 80, between 30 and 70 or between 40 and 60.

Further, the sensor element, preferably, comprises a detector for detecting a signal elicited by the detectable label. Typically, said detector being placed below the solid support and said detector being capable of selectively detecting a signal in direct proximity to said second binding agent. Depending on the detectable label, different detection techniques may be applied.

Optically detectable labels may be determined by measuring light emission, fluorescence, FRET, polarization, refraction and the like. Typical optical detectors may be photomultipliers, phototubes, ionization detectors, active-pixel sensors, phototransistors, photodiodes, quantum dot photoconductors or photodiodes, photovoltaic cells, semiconductor detectors, thermal detectors, photochemical detectors and the like.

Electrochemically detectable labels may be determined by electrode arrangements such as thin layer electrodes suitable for voltametric and, typically, amperometric measurements. The detector, typically, contains at least two electrodes of which at least one electrode is a so-called working electrode. The electrodes can be composed of all conventional electrode materials such as metals, noble metals, alloys or graphite and are preferably composed of noble metals such as gold or palladium, or graphite. The various electrodes of the sensor can be composed of the same or different materials. More typically, the electrode are made of palladium.

It will be understood that the detected presence, absence or amount of the detectable label can be subsequently transmitted to an evaluation unit. Said evaluation unit may, preferably, comprise a data processing element, such as a computer, with an implemented algorithm for determining the presence absence or amount of an anlayte in the sample based on the detected presence, absence or amount of detectable label(s).

Such an implemented algorithm may evaluate the measured signals elicited from the detectable label(s) in a complex of first binding agent, analyte and second binding agent for signal strength, signal duration and other predetermined parameters. Based on said evaluation, truly positive signals can be identified and validated and noise signals can be identified and disregarded for further evaluations. The skilled artisan is well aware of what algorithms may be used and how they can be implemented in the device of the present invention. Preferably, the formation of the complex of first binding agent, analyte and second binding agent is detected by measuring the strength and/or duration of a signal elicited by the at least one detectable label. More preferably, said signal can be detected for a predetermined characteristic time period. Characteristics of true signals are, preferably, formation within a first time window depending on one or more association constants for the complex comprising the first binding agent, analyte and the second binding agent, persistence for a predefined time window and dissociation within a second time window depending on one or more dissociation constants for the complex comprising the first binding agent, analyte and the second binding agent. Typically, a true signal can be detected for a significantly longer time than a signal elicited by at least one detectable label comprised by a freely moving first binding agent which randomly passes the anchor layer in proximity to the immobilized second binding agent. The latter one shall only generate a short noise signal.

For a precise evaluation of the signals of the detectable labels detected in accordance with the method of the present invention and for determining the analyte based thereon, computer-implemented algorithm and, in particular, artificial intelligence algorithms, machine learning algorithms and the like may be applied.

Moreover, it will be understood that even single complex formations can be determined by the method of the present invention provided that the signals from the detectable labels of an area of the solid support comprising a single or predefined amount of second binding agent can be measured. This can be achieved by using wells, beads or other predefined areas on which a single or predefined number of second binding agents can be immobilized and for which the signals from detectable labels can be determined separately. Such a separate detection is also possible by using, e.g., magneto-responsive detectors such as magnetic tunnel junctions or magnetic spin valves (see, e.g., US 5,981,297; Fernandes 2020, Nanomedicine: Nanotechnology, Biology, and Medicine 30, 102287 or Denmark 2019, Journal of Electronic Materials (48): 4749-4761), or nanopore- based detection techniques.

In step (a) according to the method of the preset invention, a sample which comprises the analyte to be determined or which is suspected to comprise the analyte to be determined is brought into contact with a biosensor as specified elsewhere herein in detail.

The term "contacting" as used herein relates to bringing the aforementioned components in physical proximity such that the first and/or second binding agent may bind to the analyte if present in the sample. It will be understood that binding of the first binding agent to the analyte and of the second binding agent to the analyte or the complex of first binding agent and analyte may require time and applying suitable conditions. The skilled person is well aware of what time is required for achieving binding and what conditions need to be applied. For example, the sample and the binding agents may be dissolved or mixed with buffers adjusting salt concentrations and/or pH value. It will be understood that suitable buffers and other auxiliary components which may be applied depend on the binding agents to be used and the chemical nature of the analyte to be determined.

In step (a) of the method of the present invention, the first binding agent and the analyte are also brought into contact to a second binding agent immobilized on the solid support which is capable of specifically binding to the analyte or to the first binding agent when bound to the analyte. Typically, the arrangement on the sensor element comprising first binding agent anchored to the anchoring layer and immobilized second binding agent is provided prior to the application of the sample, However, the sample may be brought into contact with the first binding agent and be applied to an anchor layer comprising the second binding agent immobilized on a solid support as well.

The first binding agent applied in the method of the present invention shall be either bound to a detectable label or it may be linked to a linking agent which is capable of covalently or reversibly binding the at least one detectable label.

As a result of the aforementioned activities which occur during step (a) of the method of the present invention, an analyte will be bound by the first binding agent comprising a detectable label either covalently bound thereto or reversibly bound, optionally, via a linker molecule. The said complex of analyte and first binding agent shall move within the anchor layer and shall be bound by the second binding agent immobilized on the solid support such that a complex is generated comprising the first binding agent, analyte and second binding agent ("lock-on"). Due to molecular binding kinetics, this complex will persist in proximity to the immobilization position of the second binding molecule on the solid support for a characteristic time window until it dissolves ("lock-off").

In step (b) of the method of the present invention, the aforementioned complex of first binding agent, analyte and second binding agent is detected, whereby the analyte is determined.

The term "detecting" as used herein relates to identifying the presence, absence and/or amount of label molecules covalently bound to the solid support by measuring a physical-, chemical-, physicochemical- and/or biological property of said label molecules. It will be understood that the detectable label typically generates a signal that can be measured. The signal strength and/or duration is, typically, indicative for the amount of the detectable label molecules present on the solid support. Depending on the chemical nature of the detectable label, the signal may be an active signal such as emission of light or other radiation, or it may be a passive signal, i.e. a signal which is elicited as a response to an external stimulus such as applying an electromagnetic field, radiation or others. The skilled person is well aware of how measurement of such signals elicited by the detectable label molecules can be carried out and it will be understood that the detection method to be used in accordance with the method of the present invention depend on the detectable label used. Preferred measurement methods according the present invention also include chemiluminescence measurements, fluorescence measurements, FRET measurements, electrochemiluminescence measurements, mass measurements including mass spectrometry, magnetic or electrical filed-based measurements using, e.g., ChemFETs or other electrode arrangements, radioimmunoassay measurements, dissociation-enhanced lanthanide fluoro immunoassay (DELFIA) measurements, scintillation proximity assay measurements, quantum dot technology measuremets, turbidimetry measurements or nephelometry measurements.

The detectable label may also be detected indirectly, i.e. by using a further labeled molecule that is capable of binding specifically to the immobilized detectable label on the solid support. Such a further labeled molecule may be applied to the solid support after step (a) has been carried out and prior to step (b). It will be understood that upon specific binding of one or more of said further labeled molecule to the detectable label(s) on the solid support, said further labeled molecules may be detected in step (b) by measuring a physical-, chemical-, physicochemical- and/or biological property of said labeled molecules. Yet, a further molecule that is capable of specifically binding to the immobilized detectable label may also serve as a adaptor for other labeled molecules. Suitable molecules which may serve as further labeled molecules or adaptor molecules may also be antibodies, aptamers or other molecules which allow for specific binding of a target as descried elsewhere herein. Suitable labels for such further molecules are those described for the detectable label elsewhere herein.

In the method of the present invention, first and second binding agents may be used for determining different analytes. In such a case, it will be understood that once the complexes for such different analytes are formed, each complex must possess a different detectable label. Said, different detectable labels can be, preferably, detected by using detectable a physical-, chemical-, physicochemical- and/or biological property which differs between said detectable labels.

In a particular embodiment of the method of the present invention, said solid support is a well or predetermined subdivided detection area. Preferably, each well or predetermined subdivided detection area has immobilized on it a predefined amount of second binding agent. Said method is particularly useful for determining an analyte comprises determining the amount of said analyte. Typically, said amount is determined by counting the wells or predetermined subdivided detection areas in which a complex has been formed. According to this particular embodiment, the presence or absence or the amount of analyte is detected for each well or predetermined subdivided area. Afterwards, the number of positive wells or predetermined subdivided areas shall be determined. A positive well or predetermined subdivided area shall be one which exhibits characteristic signal for a proper complex formation as described elsewhere herein having a predetermined strength or duration, e.g., a measurable signal above a predetermined strength threshold and/or over a predetermined time window. Based on the positive wells or predetermined subdivided areas and the predefined amount of second binding agent immobilized on each of said wells or predetermined subdivided areas, the amount of analyte can be determined, e.g., by calculations. For example, if each well or predetermined subdivided area contains ideally one second binding agent, the presence of one molecule of analyte in a sample shall generate one complex. Thus, if the presence of a complex in a well or on a predetermined subdivided area is determined, this presence reflects the presence of one analyte present in the sample. Thus, using a predefined amount of second binding agents on isolated items such as wells or any other predetermined subdivided areas, allows for quantitative or semi-quantitative determination of analyte molecules present in a sample. This technique is also called "digital" detection.

Advantageously, it has been found in accordance with the present invention that using a first binding agent limited in its movements to essentially two dimensions by association to an anchor layer such as a lipid layer in a sandwich assay format improves sensitivity and to suppress undesired background noise. In accordance with the present invention, an immune complex comprising a first binding agent, e.g., antibody, an analyte to be determined, a second binding agent, e.g., antibody, is formed with a certain kinetic, persists over a certain time window and is dissolved with a certain kinetic ("lock-on, lock-off" principle). Moreover, depending on the molecular configuration used, a signal of a certain strength can be expected. Thus, in the method of the present invention a kinetic measurement of complex formation and complex dissolution is determined rather than a single formation event. This allows for dynamic real-time measurements and may even make washing steps and other treatments unnecessary. Due to the use of the anchor layer, noise causing events on the solid support shall also be reduced. Depending on the nature of the detector, it is possible to essentially singularize the complex formation events. E.g., if a magneto-responsive detector or confocal detector is applied being associated with a single second binding agent initiating the complex formation in the presence of an analyte molecule, it will be understood that complex formation shall represent the said analyte molecule present in the sample. In such a digital format, the number of signals received by the individual detectors may be used to count the analyte molecules present in the sample.

Thanks to the present invention, determination of an analyte in a sample with an improved sensitivity and specificity is possible. Moreover, even single molecule events may be determined.

The present invention also relates to a device for determining an analyte suspected to be present in a sample comprising a sensor element comprising:
(i) an anchor layer which is present on a solid support;
(ii) a first binding agent which is capable of specifically binding to the analyte, which is anchored in the anchor layer and which comprises at least one detectable label; and
(iii) a second binding agent which is capable of specifically binding to the analyte when bound to the first binding agent and which is immobilized on the solid support;

The term "device" as used herein relates to a system comprising the aforementioned components operatively linked to each other as to allow the determination of the analyte according to the method of the invention.

The sensor element in accordance with the present invention may be located in a reaction zone comprised by the device. The reaction zone may either allow directly for sample application or it may be connected to a loading zone where the sample is applied. In the latter case, the sample can be actively or passively transported via the connection between the loading zone and the reaction zone to the reaction zone. Moreover, the reaction zone shall be also connected to a detector. Suitable detectors and detection techniques are described elsewhere herein in detail. The connection between reaction zone and detector shall be such that the detector can detect the detectable labels covalently bound to the solid support. Suitable connections depend on the techniques used for measuring the presence or amount of detectable label(s). For example, for optical detection, transmission of light may be required between the detector and the reaction zone while for electrochemical determination a fluidal connection may be required, e.g., between the reaction zone and an electrode. The device according to the invention, preferably, further comprises a detector for detecting a signal elicited by the detectable label, wherein said detector is placed below the solid support.

The present invention, in general relates to the use of the aforementioned device of the invention for determining an analyte suspected to be present in a sample in said sample.

Yet, the invention provides a kit for determining an analyte suspected to be present in a sample comprising the device of the present invention.

The term "kit" as used herein refers to a collection of components required for carrying out the method of the present invention including the device of the present invention. Typically, the components of the kit are provided in separate containers or within a single container. The container also typically comprises instructions for carrying out the method of the present invention. These instructions may be in the form of a manual or may be provided by a computer program code which is capable of carrying out or supports the determination of the analyte referred to in the methods of the present invention when implemented on a computer or a data processing device. The computer program code may be provided on a data storage medium or device such as an optical storage medium (e.g., a Compact Disc) or directly on a computer or data processing device or may be provided in a download format such as a link to an accessible server or cloud. Moreover, the kit may, usually, comprise analyte solutions with standardized amounts for calibration or validation or other reference amounts. The kit according to the present invention may also comprise further components which are necessary for carrying out the method of the invention such as washing solutions, solvents, and/or reagents required for detection of the detectable label. Further, it may comprise the device of the invention either in parts or in its entirety.

### FIGURES

**Figure 1****:** A schematic view of the sensor arrangement. On the left, a locked state is shown characterized by a complex of the first binding agent, analyte and second binding agent. The signal elicited by the detectable label can be measured by dark filed excitation. Such a signal shall have a characteristic lock-on, lock-off signature. On the right, causes of background noise are shown. Background noise will result from first binding agents which float in the anchor layer and which may or may not have bound to an analyte molecule already. The background signals are typically less intense and shall not persist over a significant time window.

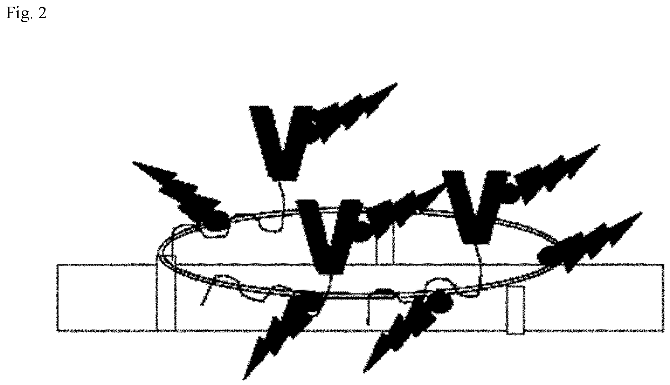

**Figure 2****:** A schematic view of a circular I-DNA molecule is shown which is anchored in the anchor layer, e.g., a lipid layer, via 3 anchoring molecules attached thereto and which contains three antibody fragments as first binding agents in accordance with the invention and attached to the said antibodies and to the circular I- DNA seven detectable labels, e.g., fluorescent labels.

## Claims

1. A method for determining an analyte suspected to be present in a sample comprising:
(a) contacting said sample with a sensor element comprising:
(i) an anchor layer which is present on a solid support;
(ii) a first binding agent which is capable of specifically binding to the analyte, which is anchored in the anchor layer and which comprises at least one detectable label; and
(iii) a second binding agent which is capable of specifically binding to the analyte when bound to the first binding agent and which is immobilized on the solid support;
for a time and under conditions which allow for specific binding of the analyte suspected to be present in the sample to the first binding agent and specific binding of the second binding agent to the analyte bound to the first binding agent; and
(b) detecting the formation of the complex of first binding agent, analyte and second binding agent whereby the analyte is determined.

2. The method of claim 1, wherein said formation of the complex of first binding agent, analyte and second binding agent is detected by measuring the strength and/or duration of a signal elicited by the at least one detectable label.

3. The method of claim 2, wherein said signal can be detected for a predetermined characteristic time period.

4. The method of any one of claims 1 to 3, wherein said anchor layer is a lipid layer or lipid bi-layer.

5. The method of any one of claims 1 to 4, wherein said first binding agent is anchored in the anchor layer via an anchoring molecule.

6. The method of any one of claims 1 to 5, wherein the amount of said first binding agent exceeds the amount of said second binding agent by a factor between 10 and 100, between 20 and 80, between 30 and 70 or between 40 and 60.

7. The method of any one of claims 1 to 6, wherein said first binding agent is selected from the group consisting of: an antibody or fragment thereof, an aptamer, a receptor molecule or fragment thereof, and a ligand molecule or fragment thereof.

8. The method of any one of claims 1 to 7, wherein said second binding agent is selected from the group consisting of: an antibody or fragment thereof, an aptamer, a receptor molecule or fragment thereof, and a ligand molecule or fragment thereof.

9. The method of any one of claims 1 to 8, wherein said determining an analyte comprises determining the presence, absence or amount of said analyte.

10. The method of any one of claims 1 to 9, wherein said detectable label is selected from the group consisting of: fluorescent labels, chemiluminescent label, radioactive labels, magnetic labels, and electrochemical labels.

11. The method of any one of claims 1 to 10, wherein said sensor element comprises a detector for detecting a signal elicited by the detectable label, said detector being placed below the solid support and said detector being capable of selectively detecting a signal in direct proximity to said second binding agent.

12. A device for determining an analyte suspected to be present in a sample comprising a sensor element comprising:
(i) an anchor layer which is present on a solid support;
(ii) a first binding agent which is capable of specifically binding to the analyte, which is anchored in the anchor layer and which comprises at least one detectable label; and
(iii) a second binding agent which is capable of specifically binding to the analyte when bound to the first binding agent and which is immobilized on the solid support.

13. The device of claim 12, wherein said device further comprises a detector for detecting a signal elicited by the detectable label, said detector being placed below the solid support.

14. Use of the device of claim 12 or 13 for determining an analyte suspected to be present in a sample in said sample.

15. A kit for determining an analyte suspected to be present in a sample comprising the device of claim 12 or 13.

## Patentansprüche

1. Verfahren zum Bestimmen eines Analyten, der vermutlich in einer Probe vorliegt, umfassend:
(a) Inkontaktbringen der Probe mit einem Sensorelement, umfassend:
(i) eine Ankerschicht, die auf einem festen Träger vorliegt;
(ii) ein erstes Bindemittel, das spezifisch an den Analyten binden kann, das in der Ankerschicht verankert ist und das mindestens eine nachweisbare Markierung umfasst; und
(iii) ein zweites Bindemittel, das spezifisch an den Analyten binden kann, wenn es an das erste Bindemittel gebunden ist, und das auf dem festen Träger immobilisiert ist;
für eine Zeit und unter Bedingungen, die spezifisches Binden des Analyten, der vermutlich in der Probe vorliegt, an das erste Bindemittel und spezifisches Binden des zweiten Bindemittels an den an das erste Bindemittel gebundenen Analyten ermöglichen; und
(b) Nachweisen der Bildung des Komplexes aus erstem Bindemittel, Analyt und zweitem Bindemittel, wodurch der Analyt bestimmt wird.

2. Verfahren nach Anspruch 1, wobei die Bildung des Komplexes aus erstem Bindemittel, Analyt und zweitem Bindemittel durch Messen der Stärke und/oder Dauer eines Signals, das von der mindestens einen nachweisbaren Markierung ausgelöst wird, nachgewiesen wird.

3. Verfahren nach Anspruch 2, wobei das Signal für einen vorbestimmten charakteristischen Zeitraum nachgewiesen werden kann.

4. Verfahren nach einem der Ansprüche 1 bis 3, wobei die Ankerschicht eine Lipidschicht oder Lipiddoppelschicht ist.

5. Verfahren nach einem der Ansprüche 1 bis 4, wobei das erste Bindemittel in der Ankerschicht über ein Ankermolekül verankert ist.

6. Verfahren nach einem der Ansprüche 1 bis 5, wobei die Menge des ersten Bindemittels die Menge des zweiten Bindemittels um einen Faktor zwischen 10 und 100, zwischen 20 und 80, zwischen 30 und 70 oder zwischen 40 und 60 überschreitet.

7. Verfahren nach einem der Ansprüche 1 bis 6, wobei das erste Bindemittel aus der Gruppe ausgewählt ist, die aus Folgendem besteht: einem Antikörper oder Fragment davon, einem Aptamer, einem Rezeptormolekül oder Fragment davon und einem Ligandenmolekül oder Fragment davon.

8. Verfahren nach einem der Ansprüche 1 bis 7, wobei das zweite Bindemittel aus der Gruppe ausgewählt ist, die aus Folgendem besteht: einem Antikörper oder Fragment davon, einem Aptamer, einem Rezeptormolekül oder Fragment davon und einem Ligandenmolekül oder Fragment davon.

9. Verfahren nach einem der Ansprüche 1 bis 8, wobei das Bestimmen eines Analyten das Bestimmen der Gegenwart, Abwesenheit oder Menge des Analyten umfasst.

10. Verfahren nach einem der Ansprüche 1 bis 9, wobei die nachweisbare Markierung aus der Gruppe ausgewählt ist, die aus Folgendem besteht: fluoreszierenden Markierungen, chemilumineszierenden Markierungen, radioaktiven Markierungen, magnetischen Markierungen und elektrochemischen Markierungen.

11. Verfahren nach einem der Ansprüche 1 bis 10, wobei das Sensorelement einen Detektor zum Nachweisen eines Signals, das von der nachweisbaren Markierung ausgelöst wird, umfasst, wobei der Detektor unter dem festen Träger platziert ist und wobei der Detektor selektiv ein Signal in direkter Nähe zu dem zweiten Bindemittel nachweisen kann.

12. Vorrichtung zum Bestimmen eines Analyten, der vermutlich in einer Probe vorliegt, umfassend ein Sensorelement, umfassend:
(i) eine Ankerschicht, die auf einem festen Träger vorliegt;
(ii) ein erstes Bindemittel, das spezifisch an den Analyten binden kann, das in der Ankerschicht verankert ist und das mindestens eine nachweisbare Markierung umfasst; und
(iii) ein zweites Bindemittel, das spezifisch an den Analyten binden kann, wenn es an das erste Bindemittel gebunden ist, und das auf dem festen Träger immobilisiert ist.

13. Vorrichtung nach Anspruch 12, wobei die Vorrichtung ferner einen Detektor zum Nachweisen eines Signals, das von der nachweisbaren Markierung ausgelöst wird, umfasst, wobei der Detektor unter dem festen Träger platziert ist.

14. Verwendung der Vorrichtung nach Anspruch 12 oder 13 zum Bestimmen eines Analyten, der vermutlich in einer Probe vorliegt, in der Probe.

15. Kit zum Bestimmen eines Analyten, der vermutlich in einer Probe vorliegt, umfassend die Vorrichtung nach Anspruch 12 oder 13.

## Revendications

1. Procédé de détermination d'un analyte suspecté d'être présent dans un échantillon comprenant :
(a) la mise en contact dudit échantillon avec un élément capteur comprenant :
(i) une couche d'ancrage qui est présente sur un support solide ;
(ii) un premier agent de liaison qui est capable de se lier spécifiquement à l'analyte, qui est ancré dans la couche d'ancrage et qui comprend au moins un marqueur détectable ; et
(iii) un second agent de liaison qui est capable de se lier spécifiquement à l'analyte lorsqu'il est lié au premier agent de liaison et qui est immobilisé sur le support solide ;
pendant une durée et dans des conditions qui permettent une liaison spécifique de l'analyte suspecté d'être présent dans l'échantillon au premier agent de liaison et une liaison spécifique du second agent de liaison à l'analyte lié au premier agent de liaison ; et
(b) la détection de la formation du complexe du premier agent de liaison, de l'analyte et du second agent de liaison moyennant quoi l'analyte est déterminé.

2. Procédé selon la revendication 1, dans lequel ladite formation du complexe du premier agent de liaison, de l'analyte et du second agent de liaison est détectée en mesurant l'intensité et/ou la durée d'un signal provoqué par l'au moins un marqueur détectable.

3. Procédé selon la revendication 2, dans lequel ledit signal peut être détecté pendant une période de temps caractéristique prédéterminée.

4. Procédé selon l'une quelconque des revendications 1 à 3, dans lequel ladite couche d'ancrage est une couche lipidique ou une bicouche lipidique.

5. Procédé selon l'une quelconque des revendications 1 à 4, dans lequel ledit premier agent de liaison est ancré dans la couche d'ancrage par l'intermédiaire d'une molécule d'ancrage.

6. Procédé selon l'une quelconque des revendications 1 à 5, dans lequel la quantité dudit premier agent de liaison dépasse la quantité dudit second agent de liaison par un facteur entre 10 et 100, entre 20 et 80, entre 30 et 70 ou entre 40 et 60.

7. Procédé selon l'une quelconque des revendications 1 à 6, dans lequel ledit premier agent de liaison est choisi dans le groupe constitué par : un anticorps ou un fragment de celui-ci, un aptamère, une molécule réceptrice ou un fragment de celle-ci et une molécule ligand ou un fragment de celle-ci.

8. Procédé selon l'une quelconque des revendications 1 à 7, dans lequel ledit second agent de liaison est choisi dans le groupe constitué par : un anticorps ou un fragment de celui-ci, un aptamère, une molécule réceptrice ou un fragment de celle-ci et une molécule ligand ou un fragment de celle-ci.

9. Procédé selon l'une quelconque des revendications 1 à 8, dans lequel ladite détermination d'un analyte comprend la détermination de la présence, de l'absence ou de la quantité dudit analyte.

10. Procédé selon l'une quelconque des revendications 1 à 9, dans lequel ledit marqueur détectable est choisi dans le groupe constitué par : les marqueurs fluorescents, un marqueur chimiluminescent, les marqueurs radioactifs, les marqueurs magnétiques et les marqueurs électrochimiques.

11. Procédé selon l'une quelconque des revendications 1 à 10, dans lequel ledit élément capteur comprend un détecteur pour détecter un signal provoqué par le marqueur détectable, ledit détecteur étant placé sous le support solide et ledit détecteur étant capable de détecter sélectivement un signal à proximité directe dudit second agent de liaison.

12. Dispositif de détermination d'un analyte suspecté d'être présent dans un échantillon comprenant un élément capteur comprenant :
(i) une couche d'ancrage qui est présente sur un support solide ;
(ii) un premier agent de liaison qui est capable de se lier spécifiquement à l'analyte, qui est ancré dans la couche d'ancrage et qui comprend au moins un marqueur détectable ; et
(iii) un second agent de liaison qui est capable de se lier spécifiquement à l'analyte lorsqu'il est lié au premier agent de liaison et qui est immobilisé sur le support solide.

13. Dispositif selon la revendication 12, dans lequel ledit dispositif comprend en outre un détecteur pour détecter un signal provoqué par le marqueur détectable, ledit détecteur étant placé sous le support solide.

14. Utilisation du dispositif selon la revendication 12 ou 13 pour déterminer un analyte suspecté d'être présent dans un échantillon dans ledit échantillon.

15. Kit de détermination d'un analyte suspecté d'être présent dans un échantillon comprenant le dispositif selon la revendication 12 ou 13.
